Europäisches Patentamt

European Patent Office

Office européen des brevets

(19)

(11) Veröffentlichungsnummer: **0 274 480**
**B1**

(12) **EUROPÄISCHE PATENTSCHRIFT**

(45) Veröffentlichungstag der Patentschrift: **23.01.91**

(51) Int. Cl.⁵: **A 61 F 9/00**

(21) Anmeldenummer: **87902390.1**

(22) Anmeldetag: **11.04.87**

(86) Internationale Anmeldenummer:
**PCT/DE87/00164**

(87) Internationale Veröffentlichungsnummer:
**WO 87/06126 22.10.87 Gazette 87/23**

(54) VORRICHTUNG ZUR KERATOTOMIE DER CORNEA.

(30) Priorität: **11.04.86 DE 3612287**

(43) Veröffentlichungstag der Anmeldung:
**20.07.88 Patentblatt 88/29**

(45) Bekanntmachung des Hinweises auf die
Patenterteilung:
**23.01.91 Patentblatt 91/04**

(84) Benannte Vertragsstaaten:
**AT BE CH DE FR GB IT LI LU NL SE**

(56) Entgegenhaltungen:
**EP-A-0 151 869**
**US-A-4 570 626**

**Ophtalmology, Band 92, Nr. 6, Juni 1985, C.A.
Puliafito et al.:"Excimer laser ablation of the
cornea and lens", Seiten 741-748 siehe
Abbildungen 1,2; Seite 742, Spalte 1, Zeilen 4-11**

(73) Patentinhaber: **AESCULAP AG**
**Möhringer Strasse 125**
**D-7200 Tuttlingen (DE)**

(72) Erfinder: **THYZEL, Reinhardt**
**Obere Bergstr. 3**
**D-8501 Heroldsberg (DE)**
Erfinder: **SCHRÖDER, Eckhard**
**Hans Sachsstr. 9**
**D-8501 Eckental (DE)**

(74) Vertreter: **Hoeger, Stellrecht & Partner**
**Uhlandstrasse 14 c**
**D-7000 Stuttgart 1 (DE)**

**Beschreibung**

Die Erfindung bezieht sich auf eine Vorrichtung zur Keratotomie der Cornea mit einem Operationslaser, dessen Strahl auf die Cornea abbildbar ist und in dessen Strahlengang eine Blendenmaske angeordnet ist, die den beleuchteten Bereich der Cornea begrenzt und die in einem Halter gehalten ist, der starr an dem Augapfel fixierbar ist.

Derartige Vorrichtungen werden beispielsweise zur sogenannten radialen Keratotomie des Auges verwendet. Eine gattungsgemäße Vorrichtung ist aus dem Artikel "EXCIMER LASER SURGERY OF THE CORNEA" von Stephen L. Trokel, R. Srinivasan und Bodil Braren, erschienen in AMERICAN JOURNAL OF OPHTALMOLOGY, 1983, S. 710—715 bekannt. Bei dieser bekannten Vorrichtung ist unmittelbar vor dem zu untersuchenden Auge eine Feldblende bzw. eine Maske mit lichtdurchlässigen Bereichen entsprechen dem gewünschten "Schnittmuster" angeordnet, die den beleuchteten Teil der Cornea begrenzt. Verwendet man einen Laser geeigneter Wellenlänge, beispielsweise einen Excimerlaser mit einer Laserwellenlänge von 193 Mikrometer aber auch einen Laser anderer Wellenlänge, beispielsweise im IR-Bereich, so wird durch das Laserlicht Gewebe abgetragen. Damit ist es möglich, "Schnitte" in der Cornea zu erzeugen, wie sie beispielsweise für die radiale Keratotomie benötigt werden. Allerdings können bei Operationen am Auge von Lebewesen Augenbewegungen nie ausgeschlossen werden. Nicht nur willkürliche oder unwillkürliche Bewegungen des Auges und Kopfbewegungen sind störend, sondern bereits Körperbewegungen durch Pulsschlag und Atmung können zu einer unkontrollierten Verbreiterung der Schnitte führen, die eine Breite im Bereich bis zu einigen 100 um haben soll. Hierbei ist zusätzlich zu berücksichtigen, daß typische Excimerlaser eine Repititionsrate von ca. 25 Schuß/sec. haben, und daß zur Erzeugung eines radialen Keratotomie-Schnittes ca. 100 sec. benötigt werden. Dadurch wird durch Augenbewegungen nicht nur der Schnitt verbreitert, sondern auch der Zusammenhang zwischen Schußzahl, Schußleistung und Schnittiefe zerstört.

Um diese Schwierigkeiten zu überwinden, ist es bekannt, auf dem Augapfel des Patienten einen Halter zu fixieren, der eine Blende mit dem Behandlungsmuster trägt. Dieser starr am Augapfel festgelegte Halter wird mit dem Bestrahlungsgerät starr verbunden, so daß auf diese Weise der Augapfel gegenüber dem Operationslaser festgehalten wird. Unwillkürliche und willkürliche Bewegungen des Augapfels sollen dadurch verhindert werden (EP-A2-0151869). Vollständig verhindern lassen sich trotzdem derartige Augapfelbewegungen nicht, insbesondere nicht unwillkürliche Bewegungen. Die Festlegung des Augapfels ist äußerst schmerzhaft, wenn derartige unwillkürliche Bewegungen des Augapfels durch den Halter gewaltsam verhindert werden, trotzdem läßt sich eine Bestrahlung der Cornea mit der gewünschten präzision nicht erreichen, da bereits kleinste Verschiebungen zu einer Verbreiterung des bestrahlten Bereiches führen.

Es ist Aufgabe der Erfindung, eine gattungsgemäße Vorrichtung anzugeben, bei der durch Augenbewegungen keine Verbreiterungen der erzeugten Schnitte auftreten.

Diese Aufgabe wird bei einer Vorrichtung der eingangs beschriebenen Art erfindungsgemäß dadurch gelöst, daß der Strahl des Operationslasers nicht nur zumindest für einen Teil des für das Licht des Operationslasers durchlässigen Bereichs beleuchtet, sondern auch den den durchlässigen Bereich umgebenden Bereich der Blendenmaske, und daß der Halter gegenüber dem Operationslaser frei beweglich ist und dadurch die Bewegung des Auges mitmachen kann.

Durch die im kennzeichnenden Teil des Patentanspruches 1 angegebenen Merkmale ist es möglich, den Einfluß von kleinen Augenbewegungen, wie sie typischerweise durch den Pulsschlag, die Atmung, willkürliche oder unwillkürliche Augen- bzw. Kopfbewegungen erzeugt werden, auszuschalten und einen scharfen und definierten Schnitt zu erzeugen, bei dem ein fester Zusammenhang zwischen Schußzahl, Schußleistung und Schnittiefe besteht:

Erfindungsgemäß ist die Blendenmaske in einem Halter gehalten, der starr am Augapfel befestigt ist, d. h. relativ zum Augapfel fixiert ist. Das Licht des Operationslasers beleuchtet sowohl den für das Licht des Operationslasers lichtdurchlässigen Bereich der Blendenmaske als auch den umgebenden Bereich. Damit wird sowohl bei Augenbewegungen während eines Laserschusses als auch zwischen zwei Schüssen immer der gleiche Bereich der Cornea mit gleichbleibender Intensität beleuchtet, so daß Schnitte erzeugt werden, deren Breite nicht unkontrolliert verändert ist, und bei denen ein definierter Zusammenhang zwischen Schußzahl, Schußleistung und Schnittiefe besteht.

Die erfindungsgemäße Vorrichtung kann dabei sowohl einen Strahlengang aufweisen, dessen Querschnitt länglich mit einer der Orientierung des Schlitzes in der Blendenmaske angepaßten Orientierung ist, als auch einen Strahlengang, der den lichtdurchlässigen Bereich der Blendenmaske abtastet.

In jedem Falle ist es jedoch vorteilhaft, wenn optische Maßnahmen vorgesehen sind, wie sie beispielsweise in den deutschen Patentanmeldungen P 35 23 340.0, 35 23 341.9 und P 35 23 342.7 beschrieben sind (Ansprüche 11 und 12).

Durch die Ausbildung des Strahlenganges des Operationslasers gemäß Anspruch 11 wird erreicht, daß das Laserlicht auf die Maske bzw. die Cornea mit einer hohen Schärfentiefe abgebildet wird, so daß sich keine Verbreiterungen des erzeugten Schnittes aufgrund der Krümmung der Cornea und hieraus resultierender unscharfer Abbildungen ergeben.

Dabei ist es möglich, den Bereich des Schnittes "flächig" zu beleuchten (Anspruch 12), oder ihn mit einer Vielzahl von "Schüssen" abzutasten.

Im übrigen wird zu Einzelheiten der in den Ansprüchen 11 und 12 beanspruchten bevorzugten Weiterbildungen des Gegenstands des Anspruches 1 auf die erwähnten Patentanmeldungen Bezug genommen.

Im Rahmen des allgemeinen Erfindungsgedankens ist es natürlich möglich, den Halter auf nahezu beliebige Weise am Augapfel zu befestigen bzw. zu fixieren. Beispielsweise ist eine Befestigung mit einer löslichen Klebstoffschicht möglich. Besonders vorteilhaft und einfach in der Anwendung ist jedoch die im Anspruch 2 gekennzeichnete Ausführung, bei der der Halter mit Blendenmaske durch Unterdruck am Augapfel fixiert ist.

In den Ansprüchen 3 und 4 sind zwei Möglichkeiten beansprucht, wie diese "starre" Verbindung durch Unterdruck realisiert werden kann:

Beispielsweise ist es möglich, in der Auflagefläche des Halters auf dem Augapfel eine Ringnut vorzusehen, und in dem von der Ringnut und Augapfel begrenzten Raum Unterdruck zu erzeugen (Anspruch 3).

Eine besonders feste Verbindung zwischen Halter und Blendenmaske einerseits und Augapfel andererseits ergibt sich bei der Lösung nach Anspruch 4, bei der in dem von Blendenmaske, Innenwand des Halters und Augapfel begrenzten Raum Unterdruck erzeugt wird. Bei dieser Ausführung "verrutscht" die Blendenmaske auch bei vergleichsweise sehr großen Augenbewegungen nicht gegenüber der Cornea.

Zum Erzeugen des Unterdrucks können nahezu beliebige Einrichtungen verwendet werden, beispielsweise die im Anspruch 5 gekennzeichnete Membranpumpe. In jedem Falle ist es jedoch vorteilhaft, wenn die Saugleistung der Pumpe etc. einstellbar ist, so daß sie individuell anpaßbar ist, und ein Kompromiß zwischen ausreichend "starrer" Verbindung und Belastung für Auge oder mechanische Teile erzielt werden kann (Anspruch 6).

Weiter ist bevorzugt, daß der Halter so ausgebildet ist, daß die Blendenmasken austauschbar sind, so daß nicht für jede Blendenmaske, mit der ein bestimmtes Schnittmuster, beispielsweise radiale Schnitte oder eine Punktfolge, erzeugt werden kann, ein eigener Halter bereitgestellt werden muß (Anspruch 9). Die Befestigung der Masken-Einsätze kann beispielsweise mittels eines Klemmringes erfolgen.

Bei einer Ausbildung des Halters gemäß Anspruch 4 ist es ausreichend, den Halter mit einem ringförmigen Anschlag zu versehen und die Blendenmaske einfach in den Halter zu legen, da der Unterdruck die Blendenmaske sicher fixiert. Diese Ausbildung erlaubt darüberhinaus ein leichtes Justieren des "Schnittmusters" relativ zur Cornea vor Erzeugen des Unterdrucks.

Die Blendenmaske, die entsprechend dem medizinisch erforderlichen Schnittmuster für das Licht des Operationslasers durchlässige und undurchlässige Bereiche aufweist, kann aus den verschiedensten Materialien gefertigt sein. Beispielsweise ist es bei Verwendung eines Excimerlasers möglich, die Maske aus Quarzglas zu fertigen, bei dem die Bereiche, die für den Laserstrahl nicht durchlässig sein sollen, mit einer Verspiegelungsschicht versehen sind.

Besonders vorteilhaft ist es jedoch, wenn die Blendenmaske gemäß Anspruch 7 in den Bereichen, in denen sie für das Licht des Operationslasers durchlässig sein soll, Durchbrüche im Blendenmaterial aufweist. Diese Ausbildung der Blendenmaske hat nicht nur den Vorteil, daß der Laserstrahl im Bereich des zu legenden Schnittes oder der Punktfolge nicht durch Reflexion oder Absorption im Blendenmaterial geschwächt wird. In Verbindung mit der Gestaltung des Halters gemäß Anspruch 4 ergibt sich der große Vorteil, daß durch den Unterdruck, der in dem von Maske, Halter und Augapfel begrenzten Raum aufrechterhalten wird, laufend Luft durch die Durchbrüche in der Maske angesaugt wird. Dieser Luftstrom kühlt die Maske, die sich eventuell durch Absorption von Laserlicht erwärmen kann. Ferner werden durch den Luftstrom gasförmige Produkte, die bei der Photoablation entstehen, vom Schnittbereich abgeführt.

In jedem Falle ist es jedoch vorteilhaft, wenn die Blendenmaske in dem für das Operationslicht undurchlässigen Bereich aus einem Material besteht, das gute Reflexionseigenschaften für das Laserlicht aufweist. Beispielsweise kann die Maske aus einem dünnen Metallblech bestehen; für UV-Operationslaser mit einer Wellenlänge von beispielsweise 193 nm ist insbesondere Aluminium als Maskenmaterial gut geeignet (Anspruch 8).

Um den Fortgang der Operation visuell beobachten zu können, ist es ferner bevorzugt, in der Blendenmaske zusätzliche Fenstersegmente vorzusehen, durch die eine Beobachtung der Cornea und insbesondere des Schnittes möglich ist (Anspruch 10).

Im Anspruch 13 ist ein bevorzugter Abstand der Maske vom Scheitel der Cornea gekennzeichnet. Bei diesem Abstand der Maske von der Cornea erfolgt keine direkte Temperaturableitung von der Maske zur Cornea; darüberhinaus können die bei der Photoablation freiwerdenden gasförmigen Produkte abziehen.

Um den gesamten Bereich der Cornea operieren zu können, ist es gemäß Anspruch 14 bevorzugt, wenn der Halter auf dem die Cornea umgebenden Teil des Augapfels aufliegt.

Ferner ist es auch möglich, in dem von der Blendenmaske, dem Halter und dem Augapfel gebildeten Raum zusätzliche Elemente wie "thermische Schilde", Strömungsstörer etc. vorzusehen (Anspruch 15).

Die Erfindung wird nachstehend anhand von Ausführungsbeispielen unter Bezugnahme auf die Zeichnung näher beschrieben, auf die im übrigen hinsichtlich aller nicht erläuterten Details ausdrücklich Bezug genommen wird. Es zeigen:

Fig. 1 einen Querschnitt durch ein erstes Ausführungsbeispiel der Erfindung, und

Fig. 2 einen Querschnitt durch ein zweites Ausführungsbeispiel der Erfindung.

In den Figuren ist mit 1 ein Halter bezeichnet, der auf dem die Cornea 2 umgebenden Teil 3 eines Augapfels 4 aufliegt. Der Halter weist einen ringförmigen Anschlag 5 auf, auf dem eine Blendenmaske 6 aufliegt, die beispielsweise aus einem dünnen Aluminiumblech besteht. In der Blendenmaske 6 befinden sich Durchbrüche 7, die entsprechend dem gewünschten Schnittmuster in der Cornea 2 angeordnet sind. Die Durchbrüche 7 können beispielsweise radiale Schlitze und/oder Punktfolgen sein.

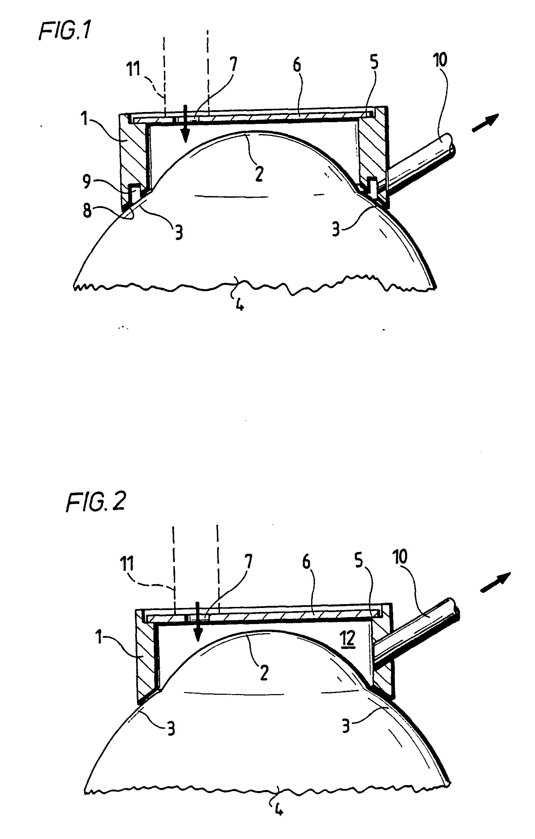

Bei dem in Fig. 1 dargestellten Ausführungsbeispiel befindet sich in der Auflagefläche 8 des Halters 1 auf dem Teil 3 des Augapfels 4 eine ringförmige Nut 9, die über einen Schlauch 10 mit einer nicht dargestellten Pumpe verbunden ist, so daß in dem von der Nut 9 und dem Augapfel 4 begrenzten Raum ein Unterdruck aufrechterhalten wird, durch den der Halter 1 mit Blendenmaske 6 am Augapfel angepreßt wird, so daß er Bewegungen des Auges "mitmacht". Damit beleuchtet ein nur schematisch dargestellter Strahl 11 eines Operationslasers, dessen Querschnitt so bemessen ist, daß er nicht nur den Durchbruch 7, sondern auch den den Durchbruch 7 umgebenden Teil der Blendenmaske 9 beleuchtet, auch bei Bewegungen des Auges immer den gleichen Bereich der Cornea. Damit ergeben sich "scharf begrenzte" Schnitte, bei denen ein fester Zusammenhang zwischen Schußzahl, Schußleistung und abgetragenem Gewebe bzw. Schnittiefe besteht.

Bei dem in Fig. 2 gezeigten zweiten Ausführungsbeispiel befindet sich im Halter 1 keine Ringnut; vielmehr wird der vom Halter 1, der Cornea 2 und der Blendenmaske 6 begrenzte Raum 12 evakuiert; hierzu ist der Raum 12 über einen Schlauch 10 mit einer nicht dargestellten Pumpe verbunden. Dieses Ausführungsbeispiel hat den Vorteil, daß die Fixierung des Halters mit Maske am Augapfel stärker ist als bei der "ringförmigen Ansaugung" des Ausführungsbeispiels gemäß Fig. 1. Darüberhinaus werden durch den ständigen Luftstrom durch die Durchbrüche 7 in den Raum 12 und über den Schlauch 10 zur Pumpe des Maskenmaterial gekühlt und gasförmige Produkte, die durch die Photoablation entstehen, abgeführt. Ferner kann die Wandstärke des Halters 1 geringer sein als bei dem Ausführungsbeispiel gemäß Fig. 1, da keine Ringnut benötigt wird.

Innerhalb des Inhalts der Patentansprüche — eine Blendenmaske relativ zur Cornea zu fixieren — sind selbstverständlich die verschiedensten Modifikationen möglich:

Beispielsweise kann die Saugleistung der Pumpe einstellbar sein, um einen guten Kompromiß zwischen Fixierung am Auge und Belastung des Auges zu erzielen. Ferner kann die Blendenmaske, die bei dem in Fig. 2 gezeigten Ausführungsbeispiel einfach in die Halterung gelegt ist, zusätzlich durch einen Sprengring befestigt sein.

## Patentansprüche

1. Vorrichtung zur Keratotomie der Cornea mit einem Operationslaser, dessen Strahl (11) auf die Cornea (2) abbildbar ist und in dessen Strahlengang eine Blendenmaske (6) angeordnet ist, die den beleuchteten Bereich der Cornea begrenzt und die in einem Halter (1) gehalten ist, der starr an dem Augapfel (4) fixierbar ist, dadurch gekennzeichnet, daß der Strahl (11) des Operationslasers nicht nur zumindest einen Teil des für das Licht des Operationslasers durchlässigen Bereichs (7), sondern auch den den durchlässigen Bereich (7) umgebenden Bereich der Blendenmaske (6) beleuchtet, und daß der Halter (1) gegenüber dem Operationslaser frei beweglich ist und dadurch die Bewegung des Augapfels (4) mitmachen kann.

2. Vorrichtung nach Anspruch 1, dadurch gekennzeichnet, daß der Halter (1) und die Blendenmaske (6) am Augapfel durch Unterdruck gehalten sind.

3. Vorrichtung nach Anspruch 2, dadurch gekennzeichnet, daß der Halter (1) im Bereich seiner Auflagefläche (8) auf dem Augapfel (4) eine Ringnut (9) aufweist, und daß eine Saugeinrichtung in der Ringnut (9) Unterdruck erzeugt.

4. Vorrichtung nach Anspruch 2, dadurch gekennzeichnet, daß eine Saugeinrichtung vorgesehen ist, die in dem vom Halter (1), der Blendenmaske (6) und der Cornea (2) begrenzten Raum (12) Unterdruck erzeugt.

5. Vorrichtung nach einem der Ansprüche 1 bis 4, dadurch gekennzeichnet, daß die Saugeinrichtung eine Membranpumpe ist.

6. Vorrichtung nach einem der Ansprüche 2 bis 5, dadurch gekennzeichnet, daß die Saugleistung der Pumpe einstellbar ist.

7. Vorrichtung nach einem der Ansprüche 1 bis 6, dadurch gekennzeichnet, daß die Blendenmaske (6) in dem Bereich, in dem sie für den Laserstrahl (11) durchlässig sein soll, Durchbrüche (17) im Maskenmaterial aufweist.

8. Vorrichtung nach Anspruch 7, dadurch gekennzeichnet, daß die Blendenmaske aus Aluminium besteht.

9. Vorrichtung nach einem der Ansprüche 1 bis 8, dadurch gekennzeichnet, daß die Blendenmaske (6) im Halter (1) austauschbar gehalten ist.

10. Vorrichtung nach einem der Ansprüche 1 bis 9, dadurch gekennzeichnet, daß in der Blendenmaske (8) zusätzliche Fenstersegmente vorgesehen sind, die während der Operation eine Beobachtung der Cornea erlauben.

11. Vorrichtung nach einem der werkegehenden Ansprüche dadurch gekennzeichnet, daß im Beleuchtungsstrahlengang der Blendenmaske zwei sammelnde optische Elemente angeordnet sind, die eine telezentrische Anordnung bilden, und zwischen denen eine weitere Blendenmaske angeordnet ist.

12. Vorrichtung nach Anspruch 10, dadurch gekennzeichnet, daß zwischen den sammelnden optischen Elementen ein optisches Drehelement angeordnet ist, das das Laserlicht auf der mit dem Augapfel verbundenen Blendenmaske dreht.

13. Vorrichtung nach einem der Ansprüche 1 bis 12, dadurch gekennzeichnet, daß der Abstand der Maske (6) vom Scheitel der Cornea (2) 1 mm bis 2 mm beträgt.

14. Vorrichtung nach einem der Ansprüche 1 bis 13, dadurch gekennzeichnet, daß der Halter (1) auf dem die Cornea (2) umgebenden Teil (3) des Auges (4) aufliegt.

15. Vorrichtung nach einem der Ansprüche 1 bis 14, dadurch gekennzeichnet, daß zusätzliche Abschirmungen in dem von der Cornea (2), dem Halter (1) und der Blendenmaske (6) begrenzten Raum (12) vorgesehen sind.

## Revendications

1. Dispositif pour la kératotomie comportant un laser chirurgical dont le faisceau (11) est reproductible sur la cornée (2) et pour lequel est disposé dans la marche du faisceau un écran (6) qui délimite la zone illuminée de la cornée et est maintenu dans un support (1) fixable de façon rigide au globe oculaire (4), caractérisé en ce que le faisceau (11) du laser chirurgical n'illumine pas uniquement une partie de la zone (7) perméable à la lumière du laser chirurgical, mais également la zone de l'écran (6) entourant la zone perméable (7) et que le support (1) est mobile par rapport au laser chirurgical et peut ainsi accompagner les mouvements du globe oculaire (4).

2. Dispositif selon la revendication 1, caractérisé en ce que le support (1) et l'écran (6) sont maintenus sur le globe oculaire par une dépression.

3. Dispositif selon la revendication 2, caractérisé en ce que le support (1) présente une rainure annulaire (9) au niveau de son assise (8) sur le globe oculaire (4) et qu'un dispositif d'aspiration crée une dépression dans la rainure annulaire (9).

4. Dispositif selon la revendication 2, caractérisé en ce qu'est prévu un dispositif d'aspiration créant une dépression dans l'espace (12) délimité par le support (1), l'écran (6) et la cornée (2).

5. Dispositif selon l'une quelconque des revendications 1 à 4, caractérisé en ce que le dispositif d'aspiration est une pompe à diaphragme.

6. Dispositif selon l'une quelconque des revendications 2 à 5, caractérisé en ce que la puissance d'aspiration de la pompe est réglable.

7. Dispositif selon l'une quelconque des revendications 1 à 6, caractérisé en ce que l'écran (6) présente des ouvertures (17) dans son matériau dans la zone dans laquelle il doit être perméable au faisceau laser (11).

8. Dispositif selon la revendication 7, caractérisé en ce que l'écran est en aluminium.

9. Dispositif selon l'une quelconque des revendications 1 à 8, caractérisé en ce que l'écran (6) est maintenu de façon interchangeable dans le support (1).

10. Dispositif selon l'une quelconque des revendications 1 à 9, caractérisé en ce que sont prévus dans l'écran (8) des segments de fenêtre supplémentaires permettant d'observer la cornée durant l'intervention.

11. Dispositif selon l'une des revendications précédentes, caractérisé en ce que sont disposés dans la marche du faisceau illuminant l'écran deux éléments optiques convergents qui forment un arrangement télécentrique et entre lesquels est disposé un autre écran.

12. Dispositif selon la revendication 10, caractérisé en ce qu'est disposé entre les éléments optiques convergents un élément optique rotatoire qui tourne la lumière laser sur l'écran relié au globe oculaire.

13. Dispositif selon l'une quelconque des revendications 1 à 12, caractérisé en ce que la distance entre l'écran (6) et le sommet de la cornée (2) est de 1 mm à 2 mm.

14. Dispositif selon l'une quelconque des revendications 1 à 13, caractérisé en ce que le support (1) repose sur la partie (3) de l'oeil (4) entourant la cornée (2).

15. Dispositif selon l'une quelconque des revendications 1 à 14, caractérisé en ce que sont prévues des protections supplémentaires dans l'espace (12) délimité par la cornée (2), le support (1) et l'écran (6).

## Claims

1. A device for keratotomy of the cornea, having a surgical laser the beam (11) of which can be imaged onto the cornea (2) and in the beam path of which there is arranged an aperture mask (6) which defines the illuminated area of the cornea and is held in a holder (1) rigidly fixable on the eyeball (4), characterized in that the beam (11) of the surgical laser illuminates not only at least part of the area (7) pervious to the light of the surgical laser but also the aperture-mask area surrounding the pervious area (7), and in that the holder (1) is freely movable in relation to the surgical laser and can thereby follow the movement of the eyeball (4).

2. A device in accordance with Claim 1, characterized in that the holder (1) and the aperture mask (6) are held on the eyeball by means of underpressure.

3. A device in accordance with Claim 2, characterized in that the holder (1) has an annular groove (9) in the area of its contact surface (8) on the eyeball (4), and in that a suction device in the annular groove (9) produces underpressure.

4. A device in accordance with Claim 2, characterized in that a suction device is provided which produces underpressure in the space (12) defined by the holder (1), the aperture mask (6) and the cornea (2).

5. A device in accordance with any one of Claims 1 to 4, characterized in that the suction device is a diaphragm pump.

6. A device in accordance with any of Claims 2 to 5, characterized in that the suction power of the pump is adjustable.

7. A device in accordance with any one of Claims 1 to 6, characterized in that the aperture mask (6) has, in the area in which it is to be pervious to the laser beam (11), openings (17) in the mask material.

8. A device in accordance with Claim 7, characterized in that the aperture mask is composed of aluminium.

9. A device in accordance with any one of Claims 1 to 8, characterized in that the aperture mask (6) is held in the holder (1) in a manner so as to be exchangeable.

10. A device in accordance with any one of Claims 1 to 9, characterized in that additional window segments are provided in the aperture mask (8), these window segments permitting observation of the cornea during the operation.

11. A device in accordance with any one of the preceding Claims characterized in that two converging, optical elements are arranged in the illuminating beam path of the aperture mask, these optical elements forming a telecentric arrangement and having a further aperture mask arranged between them.

12. A device in accordance with Claim 10, characterized in that an optical rotation-element is arranged between the converging, optical elements, the rotation element rotating the laser light on the aperture mask connected to the eyeball.

13. A device in accordance with any one of Claims 1 to 12, characterized in that the mask (6) is 1 mm to 2 mm from the apex of the cornea (2).

14. A device in accordance with any one of Claims 1 to 13, characterized in that the holder (1) rests on the part (3) of the eye (4) surrounding the cornea (2).

15. A device in accordance with any one of Claims 1 to 14, characterized in that additional shields are provided in the space (12) defined by the cornea (2), the holder (1) and the aperture mask (6).

FIG.1

FIG.2